# EUROPEAN PATENT APPLICATION

(11) **EP 2 180 043 A1**
(43) Date of publication of application: **28.04.2010**
(21) Application number: 09171868.4
(22) Date of filing: 30.09.2009
(51) Int. Cl.: C12N 5/07, C12N 5/00

(54) **Method for the differentiation of human adult stem cells into insulin-secreting cells**

(30) Priority: 23.10.2008 KR 20080104393
(71) Applicant: Kong HeeSuk, 435-1 Junggye-dong, Nowon-gu Seoul 139-859 (KR)
(72) Inventor: Haekwon, Kim, Seoul 139-859 (KR); HyunMi, Kang, Seoul 31-130 (KR)
(74) Representative: Grünecker, Kinkeldey, Stockmair & Schwanhäusser Anwaltssozietät

(57) **Abstract**

Disclosed is a method for differentiating human adult stem cells into insulin-secreting cells. Human adult stem cells, isolated from the subcutaneous adipose tissues around the eyes, are induced to differentiate into insulin-secreting cells in a medium in the presence of cytokines and growth factors including B27 supplement, fibroblast growth factor-2, epidermal growth factor, nicotinamide, glucagon-like peptide-1, activin A, insulin-like growth factor, betacellulin, etc., with a glucose shift from a high concentration to a low concentration. Having the ability to producing insulin and C-peptide at high levels, the insulin-secreting cells can be excellent curatives for type 1 diabetes mellitus.

## Description

### BACKGROUND OF THE INVENTION

### 1. Field of the Invention

The present invention relates to the induction of human adult stem cells to differentiate into insulin-secreting cells.

### 2. Description of the Related Art

Type 1 diabetes mellitus, also termed as insulin-dependent diabetes mellitus, is characterized by loss of insulin-producing beta cells, resulting generally from autoimmune attack, of the islets of Langerhans in the pancreas leading to a deficiency of insulin. Insulin plays a crucial role in homeostasis of the blood sugar level in the body. When insulin is absent or is produced in a low amount, it increases blood sugar level leading to complications in various organs such as the kidneys, nerves, retina, etc. The treatment of type 1 diabetes mellitus mostly relies on insulin use; requiring injection over the whole life of its patient, insulin use is not a fundamental cure to type 1 diabetes mellitus. On the other hand, transplantation with an exogenous pancreas or beta cells cures type 1 diabetes mellitus. This measure, however, suffers from many histological limitations due to the surgical transplantation from an exogenous pancreas. For example, the transplanted cells may be disrupted by the autoimmune reaction of the recipient. Recently, many studies have focused on the use of stem cells in the development of therapeutics for diabetes, of which embryonic and adult stem cells are representative. Studies have reported that embryonic stem cells could be *ex vivo* differentiated into insulin-producing cells which were found to cure hyperglycemia when transplanted into diabetic mice [Fujikawa T, Oh SH, Pi L, Hatch HM, Shupe T, Petersen BE (2005) Teratoma formation leads to failure of treatment for type I diabetes using embryonic stem cell-derived insulin-producing cells. Am J Pathol 166, 1781-1791; D'Amour KA, Bang AG, Eliazer S, Kelly OG, Agulnick AD, Smart NG, Moorman MA, Kroon E, Carpenter MK, Baetge EE (2006) Production of pancreatic hormone-expressing endocrine cells from human embryonic stem cells. Nat Biotechnol 24, 1392-1401]. However, embryonic stem cells have the great clinical problem of causing the generation of cancer when transplanted [Kroon E, Martinson LA, Kadoya K, Bang AG, Kelly OG, Eliazer S, Young H, Richardson M, Smart NG, Cunningham J, Agulnick AD, D'Amour KA, Carpenter MK, Baetge EE (2008) Pancreatic endoderm derived from human embryonic stem cells generates glucose-responsive insulin-secreting cells in vivo. Nat Biotechnol. 26, 397-398]. There have been reports that ectodermal progenitor cells can differentiate into insulin-producing cells [Hori Y, Gu X, Xie X, and Kim SK. (2005) Differentiation of insulin-producing cells from human neural progenitor cells. PLOS Med. 2, 103] and that pancreatic duct epithelial cells can differentiate into the same structures as those of the islets of Langerhans in the pancreas [Bonner-Weir S, Taneja M, Weir GC, .Tatarkiewicz K, Song KH, Sharma A and O'Neil JJ (2000). In vitro cultivation of human islets from expanded ductal tissue. Proc Natl Acad Sci USA. 97, 7999-8004]. The former is not advantageous in that insulin-producing cells were obtained from fetal brain cells which could induce tumor in the recipients [Amariglio N, Hirshberg A, Scheithauer BW, Cohen Y, Loewenthal R, Trakhtenbrot L, Paz N, Koren-Michowitz M, Waldman D, Leider-Trejo L, Toren A, Constantini S, Rechavi G. Donor-derived brain tumor following neural stem cell transplantation in an ataxia telangiectasia patient. PLoS Med. 2009 Feb 17;6(2): e1000029]. The latter is not advantageous in that only a very limited amount of stem cells can be obtained from ductal tissues. Further, it is reported that when embryonic stem cells are induced to differentiate in a media containing insulin, the insulin secreted from the differentiated cells is in fact the insulin taken from the media, as demonstrated by the fact that a propeptide which is split into insulin and C-peptide is not secreted [Rajagopal J, Anderson WJ, Kume S, Martinez OI, and Melton DA (2003) Insulin staining of ES cell progeny from insulin uptake. Science.299, 363; Hansson M, Tonning A, Frandsen U, Petri A, Rajagopal J, Englund MC, Heller RS, Hakansson J, Fleckner J, Skold HN, melton K, Semb H, and Serup P (2004) Artifactual insulin release from differentiated embryonic stem cells. Diabetes 53, 2603-2609]. Accordingly, many attempts have recently been made to induce the differentiation embryonic stem cells in the absence of insulin. There are reports on the differentiation of stem cells into insulin-producing cells in the presence of insulin-line growth factors or betacellulin as summarized in Table 1, below. Ethnic problems and cancer generation, as mentioned above, make it difficult to use embryonic stem cells in therapy for diabetes mellitus. Furthermore, only remarkably low levels of insulin and C-peptide result from differentiation from the pluripotent stem cells based on human skin fibroblasts or mesenchymal stem cells.

**TABLE 1**

| Studies on ex vivo Differentiation of Stem Cells into Insulin-Producing Cells | | | |
|---|---|---|---|
| Cell | Insulin secreted | C-Peptide secreted | Reference |
| Embryonic Stem Cell | - | 5ng/ug DNA | Jiang et al., 2007 ¹⁾ |
| Skin Fibroblast | | 0.15 ng/ug DNA | Tateishi et al., 2008 ²⁾ |
| Mesenchymal Stem Cell | 1.291 mU/L | 163 ng/L | Li et al., 2008 ³⁾ |

| | | | |
|---|---|---|---|
| ¹⁾ Jiang J, Au M, Lu K, Eshpeter A, Korbutt G, Fisk G, Majumdar AS (2007) Generation of insulin-producing islet-like clusters from human embryonic stem cells. Stem Cells 25, 1940-1953. ²⁾ Tateishi K, He J, Taranova O, Liang G, D'Alessio AC, Zhang Y (2008) Generation of insulin-secreting islet-like clusters from human skin fibroblasts. J Biol Chem. 283, 31601-31607. ³⁾ Li L, Li F, Qi H, Feng G, Yuan K, Deng H, Zhou H. (2008) Coexpression of Pdx1 and betacellulin in mesenchymal stem cells could promote the differentiation of nestin-positive epithelium-like progenitors and pancreatic islet-like spheroids. Stem Cells Dev.17, 815-823. | | | |

### SUMMARY OF THE INVENTION

Intensive and thorough research culminating in the present invention was conducted into a cell therapeutic for type 1 diabetes mellitus by the present inventors. This research resulted in the finding that the incubation of human adult stem cells, when made in the presence of various cytokines and growth factors known to be effective for the differentiation into beta cells in the media, and in which media the glucose concentration of the media is changed from a high level to a low one, allows the differentiation of adult stem cells into highly efficient insulin-producing cells.

Therefore, an object of the present invention is to provide a method for differentiating adult stem cells into insulin-secreting cells.

### BRIEF DESCRIPTION OF THE DRAWINGS

The above and other objects, features and other advantages of the present invention will be more clearly understood from the following detailed description taken in conjunction with the accompanying drawings, in which:

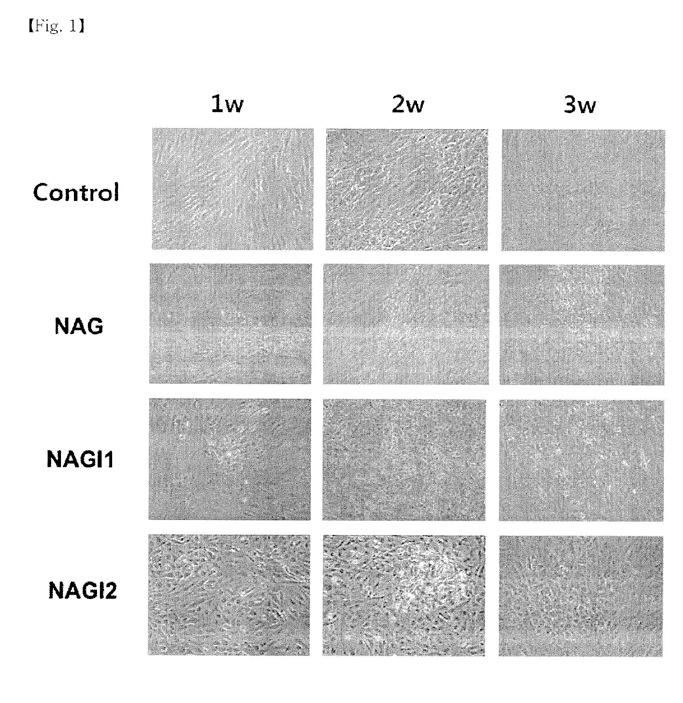

FIG. 1 is of optical microphotographs showing cell morphologies after stem cells derived from the subcutaneous layer around the eye are induced to differentiate into insulin-secreting cells for three weeks under various conditions;

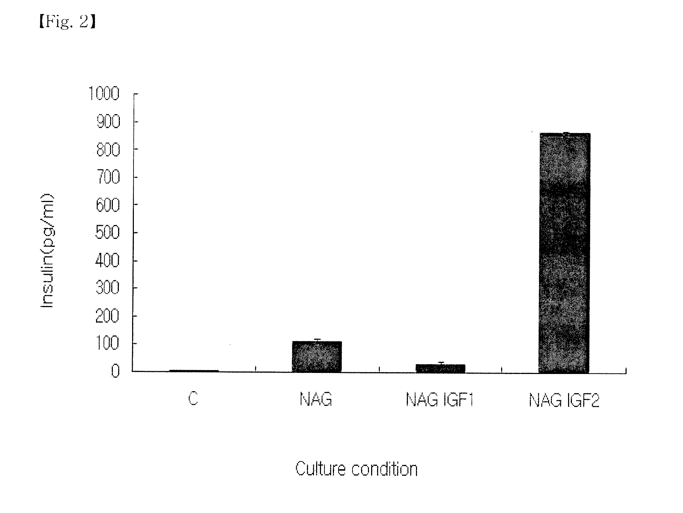

FIG. 2 is a bar graph showing the amounts of secreted insulin after stem cells derived from the subcutaneous layer around the eye were induced to differentiate into insulin-secreting cells for three weeks under various conditions and exposed for 2 hours to 25 mM glucose, as measured by an enzyme-linked immunosorbent assay;

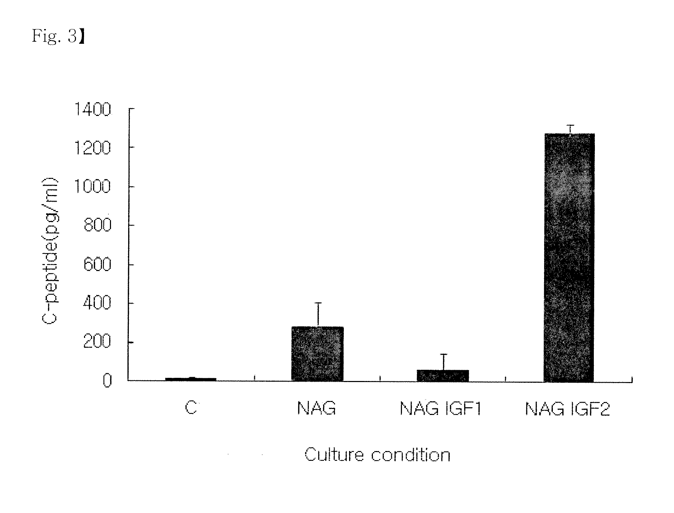

FIG. 3 is a bar graph showing C-peptide amounts secreted after stem cells derived from the subcutaneous layer around the eye were induced to differentiate into insulin-secreting cells for three weeks under various conditions and exposed for 2 hours to 25 mM glucose, as measured by an enzyme-linked immunosorbent assay ;

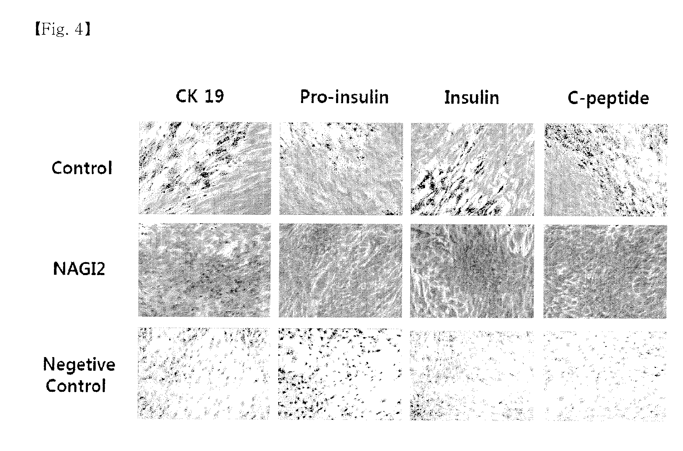

FIG. 4 is of optical microphotographs showing the expression of insulin, proinsulin, CK19 and C-peptide after stem cells derived from the subcutaneous layer around the eye were induced to differentiate into insulin-secreting cells for three weeks under various conditions, as measured by an immunocytochemical assay ;

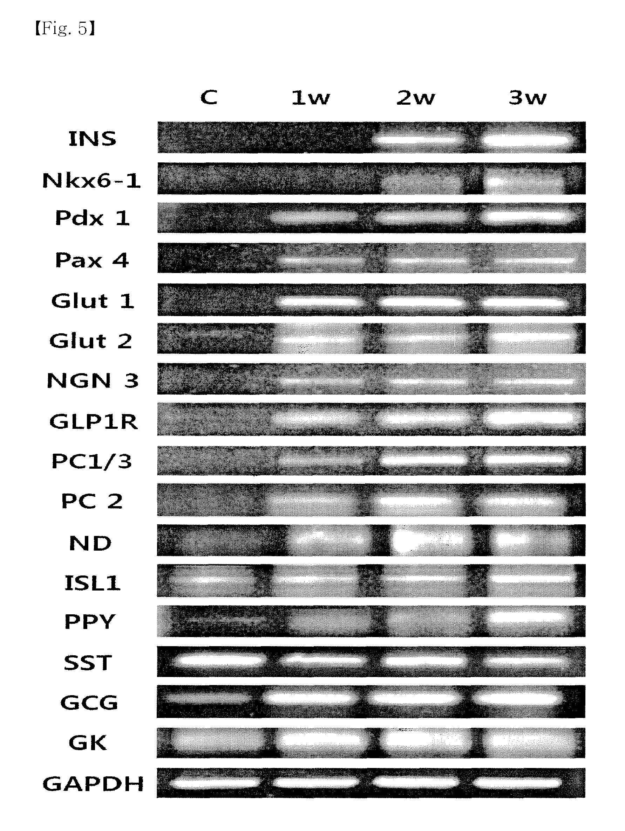

FIG. 5 is a photograph of an agarose gel showing the expression of genes characteristic of beta cells, isolated from the cells obtained after the differentiation of stem cells derived from the subcutaneous layer around the eye into insulin-secreting cells for three weeks;

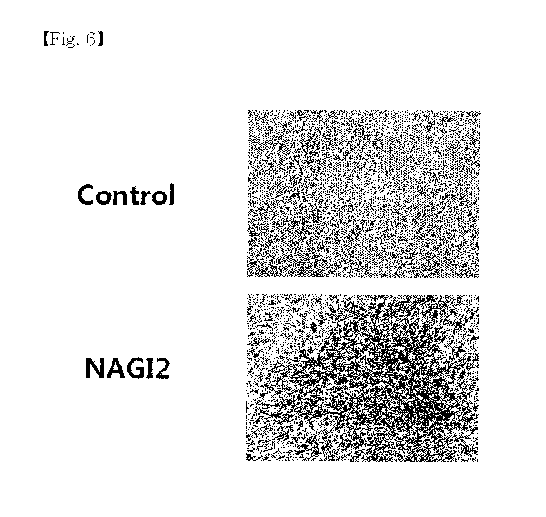

FIG. 6 is of microphotographs showing the dithizone staining, specific for insulin-producing beta cells, of the cells obtained after stem cells derived from the subcutaneous layer around the eye into insulin-secreting cells had been differentiated for three weeks;

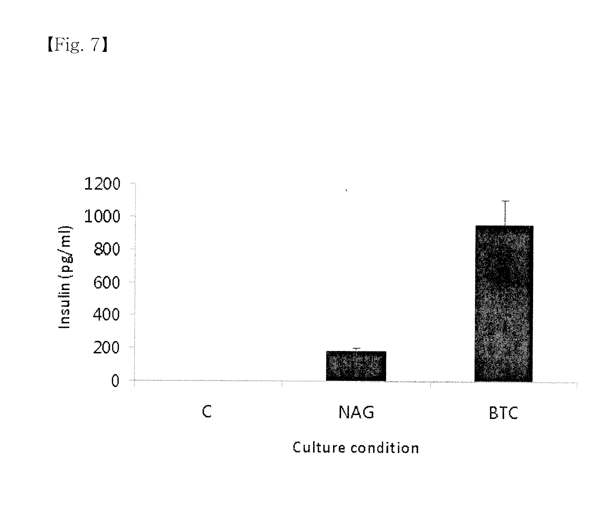

FIG. 7 is a bar graph showing the amounts of secreted insulin after stem cells derived from the subcutaneous layer around the eye were induced to differentiate into insulin-secreting cells for three weeks under various conditions and exposed for 2 hours to 25 mM glucose, as measured by an enzyme-linked immunosorbent assay ; and

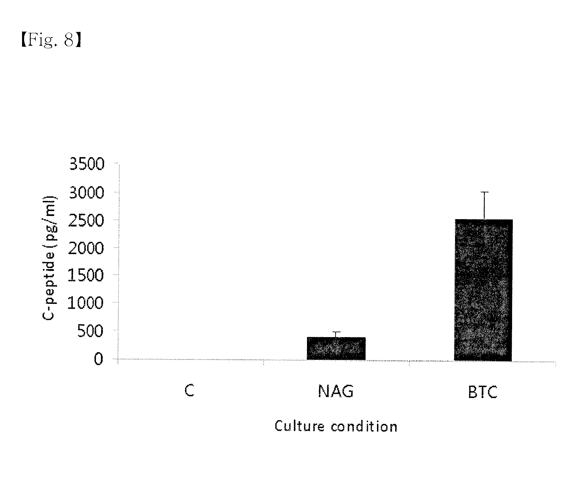

FIG. 8 is a bar graph showing amounts of secreted C-peptide after stem cells derived from the subcutaneous layer around the eye were induced to differentiate into insulin-secreting cells for three weeks under various conditions and exposed for 2 hours to 25 mM glucose, as measured by an enzyme-linked immunosorbent assay ;

### DESCRIPTION OF THE PREFERRED EMBODIMENTS

The present invention was achieved largely through two experiments: one examining the effects of insulin-like growth factors on differentiation, and another where differentiation is performed in the presence of betacellulin instead of activin A.

In accordance with one aspect thereof, the present invention provides a method for ex vivo differentiation of adult stem cells into insulin-secreting cells, comprising: culturing adult stem cells for 4 - 10 days in a medium containing 20 - 30 mM glucose, supplemented with 5 - 20% fetal bovine serum, 1 - 10 nM activin A, 5 - 20 nM glucagon-like peptide-1 and 10 - 30 ng/ml fibroblast growth factor-2, and then for 10 - 20 days in a medium containing 4 - 7 mM glucose, supplemented with 5 - 20% fetal bovine serum, 5 - 20 mM nicotinamide, 1 - 10 nM activin A, 5 - 20 nM glucagon-like peptide-1, and 10 - 100 ng/ml insulin-like growth factor.

In detail, human adult stem cells, isolated from the subcutaneous adipose tissues around the eyes, are induced to differentiate into insulin-secreting cells for the treatment of type 1 diabetes mellitus, in a medium in the presence of cytokines and growth factors including fibroblast growth factor-2, nicotinamide, a glucagon-like peptide-1, activin A, an insulin-like growth factor, etc., with a glucose shift from a high concentration for a first culture to a low concentration for a second culture.

This two-step culturing method was applied to three groups. A control was treated for 4 - 10 days with a medium containing 20 - 30 mM glucose in the presence of nicotinamide, activin A and a glucagon-like peptide-1 (NAG), followed by incubation for 10 - 20 days in a medium containing 4 - 7 mM glucose in the presence of the same cytokines [NAG group], as described in Korean Patent Application No. 2007-44663, previously filed May, 2007 by the present inventors. For a second group, its primary treatment was conducted for 4 - 10 days in a medium containing 20 - 30 mM glucose in combination with fetal bovine serum, activin A, a fibroblast growth factor-2 and a glucagon-like peptide-1 before the second incubation for 10 - 20 days in a medium containing 4 - 7 mM in combination with fetal bovine serum, nicotinamide, activin A, a glucagon-like peptide-1 and insulin-like growth factor-1 [NAGI1 group]. As for a third group, its differentiation was induced in the same manner as that of the second group, with the exception that insulin-like growth factor-2 was used instead of insulin-like growth factor-1 in the second step.

In accordance with another aspect thereof, the present invention provides a method for the ex vivo differentiation of adult stem cells into insulin-secreting cells, featuring the use of betacellulin (known to be effective for differentiation) and B27 supplement (known as a serum supplement) to establish conditions for differentiation and insulin secretion at high efficiency (Example 7).

To differentiate adult stem cells, in detail, incubation is performed for 1 - 7 days in a medium containing 20 - 30 mM glucose (high glucose medium) supplemented with B27 supplement, fibroblast growth factor-2, and an epidermal growth factor and then for 1 - 7 days in a medium with the same concentration of glucose, supplemented with fetal bovine serum, activin A, fibroblast growth factor-2 and glucagon-like peptide-1.

Afterwards, the cells are cultured for 1 - 7 days in a medium containing 4 - 7 mM glucose (low glucose medium) in the presence of cytokines and growth factors including fetal bovine serum, activin A, fibroblast growth factor-2, glucagon-like peptide-1, and finally for 10 - 20 days in a medium containing 4 - 7 mM supplemented with fetal bovine serum, betacellulin, nicotinamide and glucagon-like peptide-1.

Useful in the present invention is DMEM (Dulbecco's modified Eagle medium) or DMEM/F12 (1:1 Dulbecco's modified Eagle medium : Nutrient mixture F-12).

A detailed description will be given of the induction of adult stem cells to differentiate insulin-secreting cells, below.

The fat surgically obtained from subcutaneous layers around the eye or the cheek is treated at 37ºC for 30 min with collagenase type I, and then centrifuged. The cell pellet thus obtained is cultured in a medium supplemented with fetal bovine serum to afford adult stem cells.

For differentiation into insulin-secreting cells, the stem cells are cultured for 15 - 30 days in differentiation-inducing media. First, they are incubated for the first 4 - 10 days in a high glucose medium supplemented with 5 - 20% fetal bovine serum, 5 - 20 nM glucagon-like peptide-1, 1 - 10 nM activin A and 10 - 30 ng/ml fibroblast growth factor-2 in a collagen-coated culture dish. Thereafter, they are transferred to a low glucose medium supplemented with 5 - 20% fetal bovine serum, 5 - 20 mM nicotinamide, 5 - 20 nM glucagon-like peptide-1, 1 - 10 nM activin A, and 10 - 100 ng/ml insulin-like growth factor, followed by incubation for 10 - 20 days.

Upon completion of incubation for 15 - 30 days, the cells are treated for 10 - 15 hours with bovine serum albumin (BSA) in a low glucose medium before exposure to a high glucose medium for 2 hours. The culture thus finally obtained is quantitatively analyzed for insulin and C-peptide using an enzyme-linked immunosorbent assay. The differentiation condition defined in the present invention is found to induce the secretion of insulin and C-peptide in an improved amount as compared to the non-differentiation condition (negative control). While there were no differences in the secretion of insulin and C-peptide between the NAGI1 group (supplemented with insulin-like peptide-1) and the NAG group, the use of insulin-like peptide-2 (NAGI2 group) allowed the secretion of insulin eight times greater and C-peptide six times greater than did the use of insulin-like peptide-1 (NAGI1 group). The genetic analysis of the cells differentiated in the presence of insulin-like peptide-2 indicated that ND (neuro D1), PC (prohormone convertase)1/3, PC2, NGN3, Glut (Glucose transporter) 1, Glut 2, pax4, pdx1 -- all known as genes specific for beta cells of the pancreas -- started to be expressed when their differentiation was induced in the culture medium of the first step and that nkx 6-1 and insulin genes were expressed during the incubation in the culture medium of the second step. Also, the cells were observed to express CK19, proinsulin, insulin and C-peptide therein as measured by immunochemical staining and were strongly stained with dithizone, a dye specific for beta cells of the pancreas.

Consequently, the method for differentiating adult stem cells into insulin-secreting cells in accordance with the present invention is comprised essentially of a two-step culturing process in which the adult stem cells are induced to undergo differentiation first in a high-glucose medium in the presence of fibroblast growth factor-2, activin A and a glucagon-like peptide-1 and then in a low-glucose medium in the presence of nicotinamide, activin A, glucagon-like peptide-1 and insulin-like growth factor-2, which is superior in the efficiency of insulin secretion to that applied to the NAG group which was described in Korean Patent Application No. 2007-44663, filed on May, 2007.

Alternatively, efficient insulin-secreting cells can be differentiated from adult stem cells in accordance with the present invention as follows.

The adult stem cells are cultured for 1 - 7 days in a high-glucose medium containing 20 - 30 mM glucose, 0.5X - 4X B27 supplement, 10 - 30 ng/ml fibroblast growth factor-2 and 10 - 30 ng/ml epidermal growth factor, then for 1 - 7 days in a high-glucose medium containing 20 - 30 mM glucose glucose, 5 - 20% fetal bovine serum, 1 - 10 nM activin A, 5 - 20 nM glucagon-like peptide-1 and 10 - 30 ng/ml fibroblast growth factor-2, and then for 1 - 7 days in a low-glucose medium containing 4 - 7 mM glucose, 5 - 20% fetal bovine serum, 1 - 10 nM activin A, 5 - 15 nM glucagon-like peptide-1 and 10 - 30 ng/ml fibroblast growth factor-2, and finally for 10 - 20 days in a low-glucose medium containing 5 - 20% fetal bovine serum, 5 - 20 mM nicotinamide, 1 - 10 ng/ml betacellulin, and 5 - 20 nM glucagon-like peptide-1.

After differentiation in the presence of betacellulin, the differentiated cells (BTC) were found to produce both insulin and C-peptide each in a six times greater amount than did the NAG group.

Therefore, the cells differentiated in the presence of B27 supplement and betacellulin in lieu of fetal bovine serum and activin A, respectively, can secrete insulin more effectively than can the NAG group.

A better understanding of the present invention may be obtained through the following examples which are set forth to illustrate, but are not to be construed as the limit of the present invention.

### EXAMPLE 1: Isolation of Stem Cells

The adipose tissue, surgically excised from the subcutaneous layer around the eye in a plastic surgery hospital with the patient's permission, was treated at 37ºC for 30 min with a 0.075% collagenase type 1 solution and then with the same volume of a fetal bovine serum-supplemented medium as that of the collagenase solution to terminate the enzyme activity. After centrifugation, the cell pellet thus formed was washed twice with a culture medium and incubated in DMEM-LG supplemented with 10% fetal bovine serum.

### EXAMPLE 2: Stem Cell Culture

The stem cells obtained were cultured for 21 days in a differentiation inducing media. For this, first, the cells were seeded at a density of 5x10³ cells/well on collagen-coated 48-well plates to which a medium containing 25 mM glucose (DMEM-HG), supplemented with 10% fetal bovine serum, 4 nM activin A, 10 nM glucagon-like peptide-1 and 20 ng/ml fibroblast growth factor-2, was added before incubating for 7 days. Thereafter, the cells were further cultured for an additional 14 days in a medium containing 5.5 mM glucose (DMEM-LG), supplemented with 10% fetal bovine serum, 10 mM nicotinamide, 4 nM activin A, 10 nM glucagon-like peptide-1, and 50 ng/ml insulin-like growth factor-1 or insulin-like growth factor-2.

During incubation, the media were changed with fresh ones at regular intervals of 3 - 4 days. After incubation for a total of 3 weeks, the differentiation of the cells was examined as follows.

### EXAMPLE 3: Morphological Changes of Cells after Differentiation

Stem cells were isolated from the subcutaneous fat around the eye and cultured, and the morphological changes thereof were monitored. While remaining unchanged in morphology upon incubation in media free of growth factors and cytokines, the stem cells were observed to undergo a morphological change to a round form when they were treated with growth factors and/or cytokines such as nicotinamide, activin A, and glucagon-like peptide-1 or insulin-like growth factor-1 or -2 (FIG. 1). On week 3 after induction for differentiation, the cells became round in morphology, forming cell clusters.

### EXAMPLE 4: Examination of the Secretion of Insulin and C-Peptide

After completion of the 3-week-culture, the cells were treated for 12 hours with a low-glucose medium (DMEM-LG) supplemented with 0.5% bovine serum albumin. Then, the cells were exposed for 2 hours to a DMEM-HG and quantitatively analyzed for secreted insulin using enzyme-linked immunosorbent assay (FIG. 2). Also, the amount of C-peptide, spliced along with insulin from proinsulin, was also measured using an enzyme-linked immunosorbent assay (FIG. 3). For the enzyme-linked immunosorbent assay of both insulin and C-peptide, kits commercially available from Mercodia, Sweden, were employed. The standard solutions whose concentrations were already known and the cultures obtained above were plated in an amount of 25 µL per well onto 96-well microplates coated with human insulin or C-peptide, after which antibodies to insulin or C-peptide were added to the plates and incubated for 1 hour. Subsequently, incubation for 30 min with a TMB substrate solution was followed by absorbance measurement. Neither insulin nor C-peptide was secreted from the cells in which differentiation had not been induced while the groups which had been cultured using the two-step method of the present invention were found to produce both insulin and C-peptide. Particularly, the group cultured in the presence of insulin-like peptide-2 (NAGI2 group) was measured to secrete insulin about eight times greater and C-peptide about six times greater than did the NAG group.

### EXAMPLE 5: Expression of Proteins Specific for Insulin-Secreting Cells

After stem cells obtained from the subcutaneous fat around the eye were induced to differentiate in the presence of insulin-like peptide-2, which was proven most effective as measured by an enzyme-linked immunosorbent assay, the resulting insulin-secreting cells were assayed for the expression of CK19, insulin, C-peptide and pro-insulin (remaining unsplit before extracellular secretion) using an immunocytochemical assay (FIG. 4). After the differentiation had continued for 3 weeks, the cells were exposed for 2 hours to a high concentration of glucose, fixed at 4ºC for 90 min with 4% paraformaldehyde, and washed three times with phosphate-buffered saline. The cells were treated for 10 min with 3% hydrogen peroxide to remove endogenous peroxidase, followed by incubation at room temperature for 60 min with 2% bovine serum albumin-supplemented PBS to block background signals. The cell cultures reacted at 4ºC for 12 hours with dilutions in PBS of primary antibodies to human CK19(1:400), C-peptide(1:500), insulin(1:500) and proinsulin(1:500). For a negative control, PBS free of primary antibodies was used. They were treated for 20 min with each biotin-conjugated secondary antibody and hydrogen peroxide-conjugated streptavidin, followed by coloring with 3,3'-diaminobenzidine (DAB). Compared to the control in which differentiation was not induced, the groups treated with all nicotinamide, activin A and glucagon-like peptide were observed to have remarkably increased expression levels of all of CK19, C-peptide, insulin and proinsulin (FIG. 4).

### EXAMPLE 6: Expression of Genes Involved in Insulin Secretion

Using reverse transcription-polymerase chain reaction (RT-PCR), cells taken on Days 7, 14 and 21 after stem cells derived from the subcutaneous layer around the eye were induced to differentiate were examined for the expression of genes characteristic of insulin-secreting cells.

Initially, total RNA was isolated from the differentiated cells. One mL of Tri-reagent was added to the cells in a tube and mixed thoroughly with 200 µL of chloroform, followed by incubation at room temperature for 15 min. After centrifugation at 14,000 rpm for 15 min, the supernatant thus formed was transferred to a new tube and mixed with isopropanol. Centrifugation at 14,000 rpm for 10 min formed a pellet to which 75% ethanol was then added. This suspension was again centrifuged at 14,000 rpm for 10 min. The supernatant was drawn off and the residual 75% ethanol was evaporated, after which the residue was dissolved in sterilized, deionized water and incubated at 65ºC for 5 min to elute RNA. This RNA was quantitatively analyzed by reading absorbance at 260 nm in a UV-spectrophotometer. Total 7.5 µg of RNA was mixed with 1x reaction buffer, 1 mM dNTP, 0.5 mg/ml oligo-dT, 20U RNase inhibitor and 20U M-MuLV reverse transcriptase, and sterile deionized water was added to form a final volume of 50 µL before incubation at 42ºC for 60 min.

PCR was performed in a mixed solution containing 1x Taq buffer, 2 mM MgCl₂, 0.25U Taq polymerase and 10 pmol primers each cDNA of which sequence is complementary to each gene as shown in Table 2.

PCR began with predenaturation at 94ºC for 5 min, and 35 cycles of denaturation at 94ºC for 30 sec, annealing at respective temperatures for 30 sec and extension at 72ºC for 30 sec, and then with post-elongation at 72ºC for 5 min. Fifteen µL each of the PCR products thus obtained was loaded along with 6x loading buffer (0.25% bromophenol blue, 0.25% xylene cyanol, 40% sucrose) on 2% agarose gel and run for 30 min in the presence of an electric field of 100 V. After completion of the electrophoresis, the gels were stained with ethidium bromide and observed on a UV transilluminator (Vilber loumat, FRANCE). Expression level of each gene was determined and compared to that of glyceraldehyde-3-phosphate dehydrogenase (GAPDH) as a control. For comparative analysis, data were obtained from three measurements.

Primer sequences used in RT-PCR for the amplification of respective genes characteristic of insulin-secreting cells, their names, and sizes of the PCR products are listed in Table 2, below.

**TABLE 2**

| Gene | Primer | Size (bp) | Annealing Temp. (ºC) | Note |
|---|---|---|---|---|
| GAPDH | 5'-acaactttggtatcgtggaa-3' (SEQ ID NO. 1) | 456 | 53 | NM_002046 |
| | 5'-aaattcgttgtcataccagg-3' (SEQ ID NO. 2) | | | |
| insulin | 5'-aaccaacacctgtgcggctc-3' (SEQ ID NO. 3) | 320 | 59 | J00265 |
| | 5'-aagggctttattccatctctctcg-3' (SEQ ID NO. 4) | | | |
| ngn3 | 5'-cgtgaactccttgaactgagcag-3' (SEQ ID NO. 5) | 211 | 61 | AF234829 |
| | 5'-tggcactcctgggacgagtttc-3' (SEQ ID NO. 6) | | | |
| pdx1 | 5'-cccatggatgaagtctacg-3' (SEQ ID NO. 7) | 262 | 54 | NM_000209 |
| | 5'-gtcctcctcctttttccac-3' (SEQ ID NO. 8) | | | |
| GK | 5'-gaataccccccagagaccttttc-3' (SEQ ID NO. 9) | 182 | 61 | M90299 |
| | 5'-ggtttcttcctgagccagcg-3' (SEQ ID NO. 10) | | | |
| Is11 | 5'-agcatcaatgtcctctcaacttcc-3' (SEQ ID NO. 11) | 493 | 57 | NM_002202 |
| | 5'-tgtttggcaaggcaatgacc-3' (SEQ ID NO. 12) | | | |
| Nkx6.1 | 5'-acacgagacccactttttccg-3' (SEQ ID NO. 13) | 336 | 59 | NM_006168 |
| | 5'-tgctggacttgtgcttcttcaac-3' (SEQ ID NO. 14) | | | |
| Glut 2 | 5'-agctttgcagttggtggaat-3' (SEQ ID NO. 15) | 300 | 57 | NM_000340 |
| | 5'-aataagaatgcccgtgacga-3' (SEQ ID NO. 16) | | | |
| ND | 5'-cagaaccaggacatgccc-3' (SEQ ID NO. 17) | 216 | 60 | NM_002500 |
| | 5'-atcaaaggaagggctggtg-3' (SEQ ID NO. 18) | | | |
| Glucagon | 5'-atgaacgaggacaagcgc-3' (SEQ ID NO. 19) | 236 | 57 | NM_002054 |
| | 5'-gtaacgaaccgaccactt-3' (SEQ ID NO. 20) | | | |
| SST | 5'-ggctgcgctgtccatcgtcct g-3' (SEQ ID NO. 21) | 276 | 63 | NM_001048 |
| | 5'-ttgcgttctcggggtgccatagc-3' (SEQ ID NO. 22) | | | |
| PP | 5'-ctctgttactacagccactg-3' (SEQ ID NO. 23) | 261 | 55 | NM_002722.3 |
| | 5'-agtcgtaggagacagaaggt-3' (SEQ ID NO. 24) | | | |
| PC1/3 | 5'-ttggctgaaagagaacgggatacatct-3' (SEQ ID NO. 25) | 457 | 60 | NM_000493.3 |
| | 5'-acttctttggtgattgctttggcggtg-3' (SEQ ID NO. 26) | | | |
| PC2 | 5'-gcatcaagcacagacctacactcg-3' (SEQ ID NO. 27) | 309 | 63 | NM 003594.2 |
| | 5'-gagacacaaccacccttcatccttc-3' (SEQ ID NO. 28) | | | |
| GLUT1 | 5'-ctcactgctcaagaagacatgg-3' (SEQ ID NO. 29) | 366 | 60 | NM 006516.1 |
| | 5'-ctgggtaacagggatcaaacag-3' (SEQ ID NO. 30) | | | |
| GLP-1R | 5'-gttcccctgctgtttgttgt-3' (SEQ ID NO. 31) | 228 | 55 | NM002062.2 |
| | 5'-cttggcaagtctgcatttga-3' (SEQ ID NO. 32) | | | |
| PAX4 | 5'-cacctctctgcctgaggacacggtgag-3' (SEQ ID NO. 33) | 444 | 63 | NM 006193.1 |
| | 5'-ctgcctcattccaagccatacagtagtg-3' (SEQ ID NO. 34) | | | |

As is apparent from the data in FIG. 5, neuro D and islet 1, both known to play important roles in the development of the pancreas, and glucagon, SST(somatostatin) and PP(pancreatic polypeptide), all secreted by cells other than beta cells among the endocrine cells of the pancreas, were expressed in the cells in which differentiation was not induced, but when differentiation was induced, the cells started to express PC2, PC1/3, Glp1R, neurogenin 3, Glut2, Glut1, pax4 and pdx1 on Day 7 and Nkx6-1 and insulin on Day 14 and their expression levels were significantly increased on Day 21.

### EXAMPLE 7: Dithizone Staining

Insulin is known to complex with zinc ion (Zn⁺⁺) to form hexamers. Dithizone is a zinc-chelating agent known to selectively stain pancreatic beta cells because of their high zinc content. For this, the cells differentiated in the presence of insulin-like peptide-2 were subjected to dithizone staining. To this end, 50 mg of dithizone powder was dissolved in 5 mL of dimethyl sulfoxide (DMSO) and 10 µL of the dithizole solution was diluted in 1 mL of the culture medium. After being filtered through a 0.2 µm nylon filter, the dithizone staining solution was dropped onto the culture dish and incubated at 37ºC for 15 min. The cells were washed three times with PBS and observed under an optical microscope. As seen in FIG. 6, the differentiated cells were clearly stained, with a deep color found in the cell clusters.

In summary, as described above, when stem cells derived from the subcutaneous fat around the eye are primarily treated with a high concentration of glucose in combination with activin A, fibroblast growth factor-2 and glucagon-like peptide-1, followed by exposure to a low concentration of glucose in the presence of nicotinamide, activin A, glucagon-like peptide-1 and insulin-like growth factor-2, they differentiated into insulin-secreting cells which were observed to produce insulin, C-peptide and proinsulin in great quantity. In addition, they expressed genes characteristic of the beta cells of the pancreas as well as being clearly stained with dithizone. Consequently, the method defined according to the present invention allows the adult stem cells to effectively differentiate into insulin-secreting beta cells.

### EXAMPLE 8: Stem Cell Culture

The stem cells obtained were cultured for 21 days in a differentiation inducing media. For this, first, the cells were seeded at a density of 5x10³ cells/well on collagen-coated 48-well plates to which a medium containing 25 mM glucose (DMEM-HG), supplemented with 1X B27 supplement, 20 ng/ml fibroblast growth factor-2 and 20 ng/ml epidermal growth factor, was added before incubation for 3 days. Thereafter, the cells were further cultured for an additional 4 days in a medium containing 25 mM glucose, supplemented with 10% fetal bovine serum, 4 nM activin A, 20ng/ml fibroblast growth factor-2 and 10 nM glucagon-like peptide-1. Subsequently, the cells were cultured for 3 days in a medium containing 5.5 mM glucose (DMEM-LG), supplemented with 10% fetal bovine serum, 4 nM activin A, 20 ng/ml fibroblast growth factor-2 and 10 nM glucagon-like peptide-1, and then transferred to a new medium containing 5.5 mM glucose (DMEM-LG), supplemented with 10 mM nicotinamide, 10 nM glucagon-like peptide and 10 ng/ml betacellulin before incubation for an additional 10 days.

During incubation, the media were changed out with fresh ones at regular intervals of 3 - 4 days. After incubation for a total of 3 weeks, differentiation of the cells was examined as follows.

### EXAMPLE 9: Examination of the Secretion of Insulin and C-Peptide

After completion of the 3-week-culture, the cells were treated for 12 hours with a low-glucose medium (DMEM-LG) supplemented with 0.5% bovine serum albumin. Then, the cells were exposed for 2 hours to a DMEM-HG and quantitatively analyzed for secreted insulin using an enzyme-linked immunosorbent assay (FIG. 7). Also, the amount of C-peptide, spliced along with insulin from proinsulin, was also measured using an immunocytochemical assay (FIG. 8). For the enzyme-linked immunosorbent assay of both insulin and C-peptide, kits commercially available from Mercodia, Sweden, were employed. The standard solutions whose concentrations were already known and the cultures obtained above were plated in an amount of 25 µL per well onto 96-well microplates coated with human insulin or C-peptide, after which antibodies to insulin or C-peptide were added to the wells and incubated for 1 hour. After that, 200 µL of a TMB substrate solution was added to the wells. Following incubation for 30 min, its absorbance was measured. Neither insulin nor C-peptide was secreted from the cells in which differentiation had not been induced while the groups which had been cultured using the two-step method of the present invention were found to produce both insulin and C-peptide. Particularly, the group cultured in the same manner as in Example 8 was measured to secrete insulin about 6 times greater and C-peptide about 6 times greater than that of the NAG group.

Producing insulin and C-peptide in 7 - 8 times the amount produced by the NAG group as was disclosed in Korean Patent Application No. 2007-44663 filed on May 2007, the insulin-secreting cells differentiated from adult stem cells in a culture media according to the present invention, as described hitherto, are expected to be excellent curatives for type 1 diabetes mellitus.

Although the preferred embodiments of the present invention have been disclosed for illustrative purposes, those skilled in the art will appreciate that various modifications, additions and substitutions are possible, without departing from the scope and spirit of the invention as disclosed in the accompanying claims.

## Claims

1. A method for ex vivo differentiation of human adult stem cells into insulin-secreting cells, comprising:
culturing the adult stem cells for 4 - 10 days in a medium containing 20 - 30 mM glucose, supplemented with 5 - 20% fetal bovine serum, 1 - 10 nM activin A, 5 - 20 nM glucagon-like peptide-1 and 10 - 30 ng/ml fibroblast growth factor-2; and
culturing the cultured stem cells for 10 - 20 days in a medium containing 4 - 7 mM glucose, supplemented with 5 - 20% fetal bovine serum, 5 - 20 mM nicotinamide, 1 - 10 nM activin A, 5 - 20 nM glucagon-like peptide-1, and 10 - 100 ng/ml insulin-like growth factor.

2. The method according to claim 1, wherein the insulin-like growth factor is insulin-like growth factor-2.

3. A method for ex vivo differentiation of human adult stem cells into insulin-secreting cells, comprising sequentially culturing the adult stem cells:
for 1 - 7 days in a medium containing 20 - 30 mM glucose, supplemented with B27 supplement, 10 - 30 ng/ml fibroblast growth factor-2 and 10 - 30 ng/ml epidermal growth factor;
for 1 - 7 days in a medium containing 20 - 30 mM glucose, supplemented with 5 - 20% fetal bovine serum, 1 - 10 nM activin A, 5 - 20 nM glucagon-like peptide-1 and 10 - 30 ng/ml fibroblast growth factor-2;
for 1 - 7 days in a medium containing 4 - 7 mM glucose, supplemented with 5 - 20% fetal bovine serum, 1 - 10 nM activin A, 5 - 20 nM glucagon-like peptide-1 and 10 - 30 ng/ml fibroblast growth factor-2; and
for 10 - 20 days in a medium containing 4 - 7 mM glucose, supplemented with 5 - 20% fetal bovine serum, 5 - 20 mM nicotinamide, 1 - 10 ng/ml betacellulin and 5 - 15 nM glucagon-like peptide.


<110>	KONG, HeeSuk

<120>	Method for the differentiation of human adult stem cells into
	insulin-secreting cells

<130>	EP67303FZpau

<140>	not yet assigned
<141>	herewith

<150>	KR 10-2008-0104393
<151>	2008-10-23

<160>	34

<170>	KopatentIn 1.71

<210>	1
<211>	20
<212>	DNA
<213>	Artificial Sequence

<220>
<223>	GAPDH forward primer

<400>	1
acaactttgg tatcgtggaa	20

<210>	2
<211>	20
<212>	DNA
<213>	Artificial Sequence

<220>
<223>	GAPDH reverse primer

<400>	2
aaattcgttg tcataccagg	20

<210>	3
<211>	20
<212>	DNA
<213>	Artificial Sequence

<220>
<223>	insulin forward primer

<400>	3
aaccaacacc tgtgcggctc	20

<210>	4
<211>	24
<212>	DNA
<213>	Artificial Sequence

<220>
<223>	insulin reverse primer

<400>	4
aagggcttta ttccatctct ctcg	24

<210>	5
<211>	23
<212>	DNA
<213>	Artificial Sequence

<220>
<223>	ngn3 forward primer

<400>	5
cgtgaactcc ttgaactgag cag	23

<210>	6
<211>	22
<212>	DNA
<213>	Artificial Sequence

<220>
<223>	ngn3 reverse primer

<400>	6
tggcactcct gggacgagtt tc	22

<210>	7
<211>	19
<212>	DNA
<213>	Artificial Sequence

<220>
<223>	pdx1 forward primer

<400>	7
cccatggatg aagtctacg	19

<210>	8
<211>	19
<212>	DNA
<213>	Artificial Sequence

<220>
<223>	pdx1 reverse primer

<400>	8
gtcctcctcc tttttccac	19

<210>	9
<211>	23
<212>	DNA
<213>	Artificial Sequence

<220>
<223>	Glucokinase forward primer

<400>	9
   gaataccccc cagagacctt ttc	23

<210>	10
<211>	20

<212>    DNA
<213>    Artificial Sequence

<220>
<223>    Glucokinase reverse primer


<400>    10
ggtttcttcc tgagccagcg                                                    20


<210>    11
<211>    24
<212>    DNA
<213>    Artificial Sequence

<220>
<223>    Isl1 forward primer


<400>    11
agcatcaatg tcctctcaac ttcc                                               24


<210>    12
<211>    20
<212>    DNA
<213>    Artificial Sequence

<220>
<223>    Isl1 reverse primer


<400>    12
tgtttggcaa ggcaatgacc                                                    20


<210>    13
<211>    21
<212>    DNA
<213>    Artificial Sequence

<220>
<223>    Nkx6.1 forward primer


<400>    13
acacgagacc cactttttcc g                                                  21


<210>    14
<211>    23
<212>    DNA
<213>    Artificial Sequence

<220>
<223>    Nkx6.1 reverse primer


<400>    14
tgctggactt gtgcttcttc aac                                                23


<210>    15
<211>    20
<212>    DNA
<213>    Artificial Sequence

<220>
<223>    Glut 2 forward primer

<400>    15
agctttgcag ttggtggaat                                                    20

<210>    16
<211>    20
<212>    DNA
<213>    Artificial Sequence

<220>
<223>    Glut 2 reverse primer

<400>    16
aataagaatg cccgtgacga                                                    20

<210>    17
<211>    18
<212>    DNA
<213>    Artificial Sequence

<220>
<223>    ND forward primer

<400>    17
cagaaccagg acatgccc                                                      18

<210>    18
<211>    19
<212>    DNA
<213>    Artificial Sequence

<220>
<223>    ND reverse primer

<400>    18
atcaaaggaa gggctggtg                                                     19

<210>    19
<211>    19
<212>    DNA
<213>    Artificial Sequence

<220>
<223>    Glucagon forward primer

<400>    19
atcaaaggaa gggctggtg                                                     19

<210>    20
<211>    18
<212>    DNA
<213>    Artificial Sequence

<220>
<223>    Glucagon reverse primer

<400>    20
gtaacgaacc gaccactt                                                    18

<210>    21
<211>    22
<212>    DNA
<213>    Artificial Sequence

<220>
<223>    SST forward primer

<400>    21
ggctgcgctg tccatcgtcc tg                                               22

<210>    22
<211>    23
<212>    DNA
<213>    Artificial Sequence

<220>
<223>    SST reverse primer

<400>    22
ttgcgttctc ggggtgccat agc                                              23

<210>    23
<211>    20
<212>    DNA
<213>    Artificial Sequence

<220>
<223>    PP forward primer

<400>    23
ctctgttact acagccactg                                                  20

<210>    24
<211>    20
<212>    DNA
<213>    Artificial Sequence

<220>
<223>    PP reverse primer

<400>    24
agtcgtagga gacagaaggt                                                  20

<210>    25
<211>    27
<212>    DNA
<213>    Artificial Sequence

<220>
<223>    PC1/3 fprward primer

<400>    25
ttggctgaaa gagaacggga tacatct                                          27

<210> 26
<211> 27
<212> DNA
<213> Artificial Sequence

<220>
<223> PC1/3 reverse primer


<400> 26
acttctttgg tgattgcttt ggcggtg 27


<210> 27
<211> 24
<212> DNA
<213> Artificial Sequence

<220>
<223> PC2 forward primer


<400> 27
gcatcaagca cagacctaca ctcg 24


<210> 28
<211> 25
<212> DNA
<213> Artificial Sequence

<220>
<223> PC2 reverse primer


<400> 28
gagacacaac cacccttcat ccttc 25


<210> 29
<211> 22
<212> DNA
<213> Artificial Sequence

<220>
<223> GLUT1 forward primer


<400> 29
ctcactgctc aagaagacat gg 22


<210> 30
<211> 22
<212> DNA
<213> Artificial Sequence

<220>
<223> GLUT1 reverse primer


<400> 30
ctgggtaaca gggatcaaac ag 22


<210> 31

<211>    20
<212>    DNA
<213>    Artificial Sequence

<220>
<223>    GLP-1R forward primer


<400>    31
gttcccctgc tgtttgttgt                                                    20


<210>    32
<211>    20
<212>    DNA
<213>    Artificial Sequence

<220>
<223>    GLP-1R reverse primer


<400>    32
cttggcaagt ctgcatttga                                                    20


<210>    33
<211>    27
<212>    DNA
<213>    Artificial Sequence

<220>
<223>    PAX4 forward primer


<400>    33
cacctctctg cctgaggaca cggtgag                                            27


<210>    34
<211>    28
<212>    DNA
<213>    Artificial Sequence

<220>
<223>    PAX4 reverse primer


<400>    34
ctgcctcatt ccaagccata cagtagtg                                           28